# EUROPEAN PATENT APPLICATION

(11) **EP 0 691 111 A2**
(43) Date of publication of application: **10.01.1996**
(21) Application number: 95110219.3
(22) Date of filing: 30.06.1995
(51) Int. Cl.: A61F 7/00

(54) **A thermal compress**

(30) Priority: 01.07.1994 GB 9413332
(71) Applicant: Taylor, Alan Keith, Middlewich, Cheshire CW10 9HT (GB)
(72) Inventor: Taylor, Alan Keith, Middlewich, Cheshire CW10 9HT (GB)
(74) Representative: Walters, Frederick James

(57) **Abstract**

A thermal compress 10 has a pad 11 with two sets of straps 30 and 32 for securing the pad to the limb of an animal. The pad 11 is divided into an array of flexibly interconnected sealed pockets 20 containing a synthetic foam core which is impregnated with a fluid having a freezing point below 0°C.

A method of manufacturing a thermal compress 10 is described.

## Description

The present invention relates to a thermal compress primarily developed for attachment to an injured limb, or body, of an animal (or human) to aid recovery of the injury.

Thermal compresses of this type are well known in the art and generally comprise a flexible pad containing a fluid medium which has a freezing point below 0°C and which remains malleable at low temperatures of, for example, -10°C. Such compresses are usually cooled and secured about the region of an injury by straps, where the cool pad is used to reduce the temperature in the region of injury and help reduce swelling. Examples of such thermal compresses include US Patent No. 3822705 (Pilotte) and GB Patent No. 1345261 (Faint). However, the known compresses fail to address the problems caused by the effects of gravity and flexibility of the pad and movement of the injured limb in an inexpensive and safe manner which enables a simple construction.

Primarily, compresses employing a liquid coolant tend to have an uneven temperature distribution since the effect of gravity and flexibility of the pad will tend to draw the liquid downwardly to collect at a lower point of the pad. Alternatively, where gels are used as a coolant these prove expensive and complex to produce and may reduce the flexibility of the pad. In addition, the constituents of such gels or highly viscous fluids, if used, may prove harmful if they leak from the pad and enter the body of the patient.

It is an object of the present invention to provide a thermal compress which alleviates the aforementioned problems in a relatively simple and inexpensive manner.

According to the present invention there is provided a thermal compress having a pad for application to a limb or body of an animal, which pad comprises an array of at least two flexibly interconnected sealed pockets of flexible sheet plastics material, each pocket containing an open structured synthetic foam core which is impregnated with a fluid having a freezing point below 0°C. Preferably, each foam core will be saturated with the fluid.

In use, the foam in each pocket will alleviate the drainage of the fluid downwardly in the pad, thus maintaining a more even distribution of fluid across the pad. The pad itself will be cooled to a temperature below 0°C and the even distribution of the fluid in the pad will allow a more even temperature distribution across the pad, increasing the effectiveness of the compress by enabling the cooled pad to be positioned correctly relative to an injured site.

Preferably, the array of pockets extends in more than one direction and, usually, the pad will be rectangular in shape having longitudinally extending parallel edges with the array of individual pockets extending both longitudinally and transversely. Alternatively, the array of individual pockets may extend diagonally with respect to the longitudinal edges of the pad. The use of such arrays of individual pockets helps maintain a more even fluid distribution across the pad since any downward drainage effect of the fluid is alleviated by the limitation of each pocket. Furthermore, the use of individual pockets provides more flexible seams across the pad allowing a greater degree of flexibility in the pad along such seams.

Preferably, the fluid used in the pockets will be an aqueous solution of alcohol, usually consisting of 70% water and 30% alcohol since this solution is non-toxic and has a low freezing point, below -20°C. The sheet plastics material used will usually comprise polyurethane.

The thermal compress will usually comprise securing means for securing the pad in the required position on a limb or body and this securing means will preferably comprise at least one strap and fastening means for engaging the strap, both connected to the pad.

In addition, the thermal compress may comprise several pads joined together.

Further according to the present invention there is provided a method of manufacturing a thermal compress, comprising the steps of substantially heat sealing a pre-shaped sheet of open structured synthetic foam between two opposed sheets of flexible plastics material to form a pad, which pad is divided into an array of at least two individual pockets by compressing and heat sealing the plastics material and foam therebetween along flexible seams across the pad to define the individual pockets, leaving access openings through the plastics material communicating between the exterior of the pad and each pocket; injecting a fluid having a freezing point below 0°C through each access opening into a foam core of each pocket to impregnate said foam and then heat sealing each access opening.

A thermal compress constructed in accordance with the present invention will now be described, by way of reference only, with reference to the accompanying illustrative drawings in which:-
Figure 1 shows a thermal compress in accordance with the present invention designed for treatment of an injured limb:
Figure 2 shows a cross section of the compress of Figure 1 along the line II-II;
Figure 3 shows the compress of Figure 1 when secured to the forelimb of a horse; and
Figure 4 shows a further embodiment of a thermal compress in accordance with the present invention.

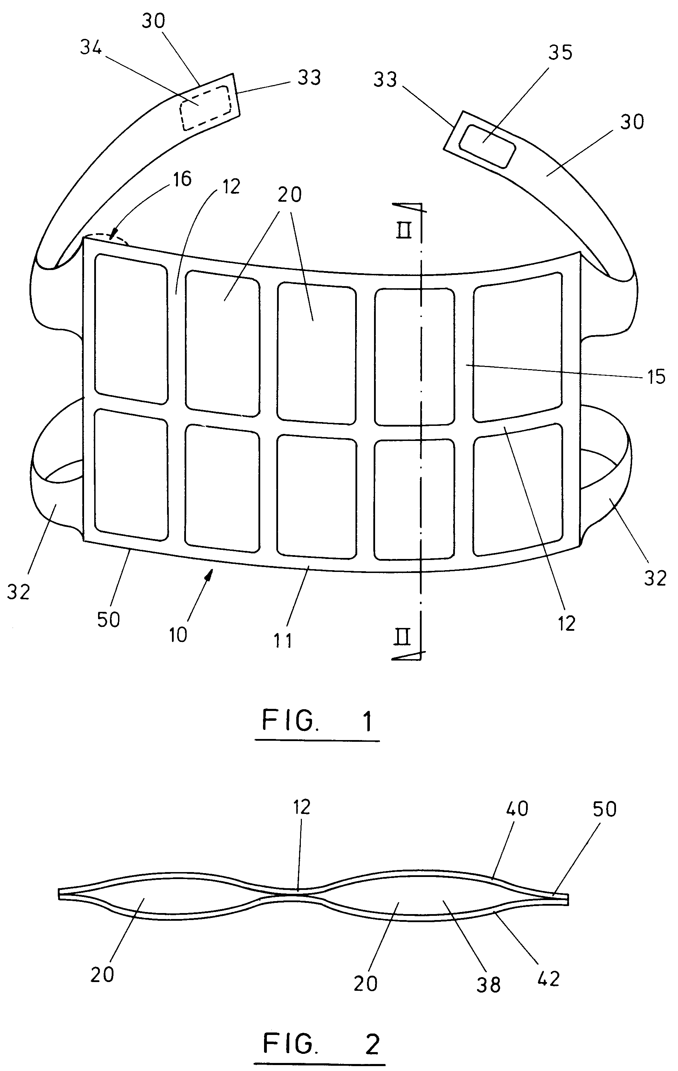

A thermal compress 10 (Figure 1) comprises a pad 11 and two sets of straps 30 and 32. Each set of straps 30 and 32 have connected to their free ends 33 fixing means 34 and 35. In this example the fixing means comprises a set of engaging velcrose pads which may be simply contact engaged. However, it will be appreciated that other securing methods could just as easily be employed such as a conventional buckle arrangement between each set of straps.

The pad 11 consists of a layer of pre-shaped, open structured synthetic foam 38 sandwiched between two opposed sheets 40, 42 of plastics material (polyurethane) (Figure 2). The polyurethane sheets 40, 42 are greater in size than the foam 38 and the overlap of the polyurethane sheets are substantially heat sealed around their peripheral edges 50. However, during this heat sealing process, several access openings between the polyurethane sheets 40, and 42 are left about the periphery of the pad 11, allowing access to the foam 38. One such opening is shown for, illustrative purposes only, at 16 in Figure 1.

The pad 11 is then divided into an array of individual pockets 20, each having a foam core 38, by compressing the pad (and therefore the foam) along a series of predetermined lines and producing heat sealed seams 12 along these lines between the two sheets of polyurethane. These seams 12 effectively divide the pad into isolated pockets 12.

Each of the access openings 16 left by the initial heat sealing process of the periphery of the sheets 40 and 42 are designed to coincide with one of the pockets 12, such that access is provided to the foam core 38 of each pocket 12. Metered and controlled amounts of a 30% alcohol, 70% water solution are then injected through each access opening under carefully controlled conditions, allowing natural expansion of the compressed foam to saturate the foam cores 38 of each pocket 12. Once the foam in each pocket 12 has been saturated the access openings are heat sealed to leave airtight pockets having saturated foam cores 38.

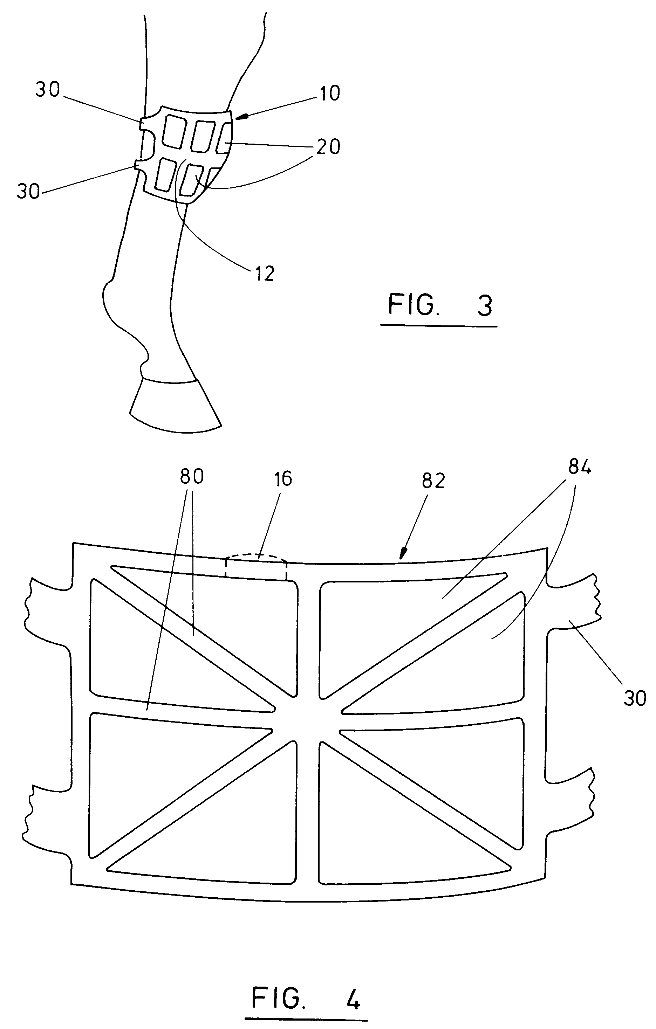

In use, the thermal compress is cooled, usually in a freezer compartment, to temperatures approaching -20°C at which temperatures the aqueous alcohol solution still remains flexible. The cooled, flexible pad is then placed or wrapped about the site of the injury, such as a forelimb of a horse (Figure 3), and held in position by the straps 30, 32. The cooled pad then draws heat from the region of the injury and helps reduce swelling. It is well known to medics and veterinarians that early removal of heat from an injury speeds up the recovery process.

The more seams employed by the pad enable an increased flexibility of the pad and the use of seams in more than one direction enables greater flexibility in more than one direction. This is particularly advantageous when the pad is used on joints whereby the seams in one direction allow the pad to be wrapped around a limb and the seams extending in a transverse direction enable the pad to flex with the movement of the joint, thus alleviating possible restriction to movement that existing gaiters may cause. This is obviously an important feature since it is desirable for such gaiters not to incapacitate the injured party, be it animal or human, but to allow relatively free movement by the wearer when it is in use.

Although there are many known fluids having a freezing point below 0°C many of these are found to be toxic, the obvious example being glycol. The obvious drawbacks of toxic fluids arise where such thermal compresses are used on animals which may tend to worry and gnaw at such compresses which may subsequently be damaged and leak. Therefore, non-toxic fluids are beneficial since they will not harm the animal and, through research, a 30% alcohol, 70% water mixture has proved to be most suitable, incorporating the minimum amount of alcohol that can prevent freezing of the water at -20°C, being non-toxic, having a low freezing point and being relatively inexpensive.
It was found necessary to use an alcohol/water solution (rather than pure alcohol) to reduce the flammability of the fluid, especially since heat sealing techniques are used in production.

It will be appreciated that the present invention is not limited to the particular embodiment shown in Figure 1. The size and shape of the pad can be varied for different requirements such as which animal it is to be used for, what part of the body etc. For example, the pad can be designed to completely encompass a limb. Also, the array of pockets 12 can be varied for different requirements. Figure 4 shows a thermal compress having a rectangular pad 82 with seams 80 extending diagonally with respect to the periphery of the pad 82 as well as parallel to the edges of the pad, creating triangular pockets 84.

In addition, it will also be possible to produce pads according to the present invention whereby the fluid is introduced into the pockets of the pad through access holes through the surface of the plastics sheets and which access holes may be heat sealed after the foam core is saturated. One means for doing this would be to provide one of the two plastics sheets with preformed apertures to coincide with each pocket during manufacture and which apertures could be heat sealed when required. This would obviously allow a larger number of pockets to be incorporated in the pad. Still further, another possible method of construction would be to saturate the foam layer in the pad before the seams are formed, thus obviously reducing the number of access holes required. However, when using the alcohol solution it is found to be more beneficial to insert the solution after the seams are formed due to the increased risk of fire if heat sealing were to take place through alcohol soaked foam. Obviously, if non flammable fluids were used then it would be possible to soak the foam before heat sealing the seams.

Also it is possible to interconnect several smaller pads produced in accordance with the present invention to increase the size of the thermal compress.

## Claims

1. A thermal compress having a pad for application to a limb or body of an animal, comprising an array of at least two flexibly interconnected sealed pockets of flexible sheet plastics material, each said pocket containing an open structured synthetic foam core which is impregnated with a fluid having a freezing point less than 0°C.

2. A thermal compress as claimed in claim 1 in which the foam core is saturated with said fluid.

3. A thermal compress as claimed in either of the preceding claims in which said pockets are interconnected by flexible strips between adjacent pockets.

4. A thermal compress as claimed in claim 3 in which said flexible strips extend in more than one direction in the plane of the pad so that said array of pockets extend in more than one direction in the plane of the pad.

5. A thermal compress as claimed in any one of the preceding claims in which said pad is rectangular in shape having longitudinally extending parallel edges with the array of individual pockets extending both longitudinally and transversely in the plane of the pad.

6. A thermal compress as claimed in any one of claims 1 to 4 in which said pad is rectangular in shape having longitudinally extending parallel edges with said array of individual pockets extending diagonally in the plane of the pad with respect to the longitudinally extending edges of the pad.

7. A thermal compress as claimed in any one of the preceding claims in which the fluid comprises an aqueous solution of alcohol.

8. A thermal compress as claimed in claim 7 in which said aqueous solution comprises 70% water and 30% alcohol.

9. A thermal compress as claimed in any one of the preceding claims in which said sheet plastics material comprises polyurethane.

10. A thermal compress as claimed in any one of the preceding claims further comprising securing means for securing the pad in a required position on a limb or body.

11. A thermal compress as claimed in claim 10 in which said securing means comprises at least one strap and fastening means for engaging said strap and fastening means being connected to said pad.

12. A thermal compress as claimed in any one of the preceding claims comprising an array of several said pads connected together.

13. A method of manufacturing a thermal compress comprising the steps of substantially heat sealing a pre-shaped sheet of opened structured synthetic foam between two opposed sheets of flexible plastic material to form a pad, dividing said pad into an array of at least two individual pockets by compressing and heat sealing the plastic materials and foam therebetween along flexible seams of the pad to define said individual pockets, providing access opening through the said plastics material communicating between the exterior of the pad and each pocket and injecting a fluid, having a freezing point less than 0°C, through each access opening into a foam core of each pocket to impregnate said foam and then heat sealing each access opening.
